# EUROPEAN PATENT SPECIFICATION

(11) **EP 1 411 100 B1**
(45) Date of publication and mention of the grant of the patent: **11.06.2008**
(21) Application number: 03023183.1
(22) Date of filing: 13.10.2003
(51) Int. Cl.: C09J 153/02, A61L 15/58, C09J 7/02

(54) **Hotmelt adhesive and disposable product using the same**
Heissschmelzkleber und diesen verwendender Wegwerfgegenstand
Adhésif thermofusible et produit jetable utilisant celui-ci

(30) Priority: 15.10.2002 JP 2002300607
(43) Date of publication of application: 21.04.2004
(73) Proprietor: Nippon NSC Ltd., Osaka 562 (JP)
(72) Inventor: Maeda, Neohiro, Itami-shi Hyogo 664-0874 (JP); Sado, Masashi, Toyonaka-shi Osaka 560-0084 (JP); Kurdoa, Hisashi, Kawanishi-shi Hyogo 666-0122 (JP)
(74) Representative: Held, Stephan

(56) References cited:
- EP-A- 0 744 178
- WO-A-02/00805
- WO-A-96/11236
- US-A- 5 342 685
- US-B1- 6 277 488

## Description

### BACKGROUND OF THE INVENTION

### 1. Field of the Invention

The present invention relates to a method of obtaining a disposable product comprising applying a hot melt adhesive, wherein the hot melt adhesive is preferably used to attach a polyolefin film constituting the disposable product such as paper diapers, sanitary napkins, pet sheets, hospital gowns or sanitary gowns for operation and another member such as nonwoven fabrics, water absorbing paper or elastic members, and to a disposable product obtained by said method.

### DESCRIPTION OF THE PRIOR ART

Hotmelt adhesives based on styrene block copolymers are widely used for disposable products typified by paper diapers and napkins. For example, paper diapers are produced by attaching a polyethylene film and another member (e.g., nonwoven fabric, an elastic member such as natural rubber, and water-absorbing paper or the like) with a hotmelt adhesive. The hotmelt adhesive can be applied to various constituent members using various methods, but in any method, the hotmelt adhesive is heated and melted so that the viscosity is appropriate and applied to various constituent members in the form of dots, lines, stripes, spirals, planes or other shapes. In recent years, a method of applying the hotmelt adhesive by spraying, which allows attachment in a wide range with a small amount, is commonly used to apply the hotmelt adhesive.

At present, regarding paper diapers, there is a demand for good touch, and it has been considered to reduce the thickness of the polyethylene film or various members such as nonwoven fabric to improve the flexibility and the touch of paper diapers. The material cost can be significantly reduced by reducing the thickness of the various members. However, when the polyethylene film becomes thin, the heat resistance is significantly reduced, and when the hotmelt adhesive is applied at a temperature of 150°C or more, the polyethylene film is melted or lines are formed in the polyethylene film. Therefore, there is a demand for a hotmelt adhesive that can be applied at a lower temperature, for example, at a temperature of 130 °C or less, that is, a low temperature applicable hotmelt adhesive. Furthermore, there is a demand for a hotmelt adhesive that can be applied at a lower temperature and can be applied by spraying.

Also, global environmental protection such as not producing waste materials and reducing power consumption has been taken into consideration when producing paper diapers. Taking this into consideration, for example, a method can be used in which a produced hotmelt adhesive is placed in a container that can store the hotmelt adhesive while keeping the temperature of the hotmelt adhesive constant and can be supplied in a liquid state.

Furthermore, in view of the workability and the environmental protection when applying the hotmelt adhesive, it has been required to reduce the viscosity of the hotmelt adhesive. The hotmelt adhesive generally includes a base polymer and a plasticizer, and it has been considered to obtain a hotmelt adhesive whose viscosity has been reduced by reducing the amount of the base polymer and increasing the amount of the plasticizer. However, when paper diapers are produced using a low viscous hotmelt adhesive produced by these methods, the balance between the adhesion and the holding power (cohesion) with respect to a polyethylene film and a polypropylene film constituting the member of paper diapers is reduced, and the softening point becomes too low. Therefore, there is a problem such as poor adhesion because a solidified hotmelt adhesive stored in a warehouse is melted in summer and cannot maintain the form (that is, cold flow phenomenon).

The conventional hotmelt adhesives include a styrene-isoprene-styrene block copolymer (hereinafter, also referred to as "SIS") as a base polymer to reduce the amount of the base polymer and increase the amount of a plasticizer so as to reduce the viscosity, and each hotmelt adhesive includes a specific SIS as an essential component (e.g., see Japanese Laid-Open Patent Publication (Tokkai) No. 4-35169 (pages 2-4), Japanese Laid-Open Patent Publication (Tokkai) No. 5-78637 (pages 2-4), Laid-Open Patent Publication (Tokuhyo) No. 4-501285 (pages 1-3), Japanese Laid-Open Patent Publication (Tokuhyo) No. 2001-525446 (pages 2-8), and Japanese Laid-Open Patent Publication (Tokkai) No. 8-333563 (pages 2-3). However, in any of the hotmelt adhesives, the characteristics can be insufficient, for example, the adhesion and the holding power to the paper diaper can be insufficient, the viscosity is not sufficiently reduced when the softening point is set to a predetermined value, or the softening point becomes too low when the viscosity is reduced. Since any of the hotmelt adhesives contains SIS, the heat stability is insufficient, and if it is stored in the form of a liquid or a semi-liquid and supplied, the originally designed performance cannot be obtained because of the deterioration during the storage.

Furthermore, the conventional hotmelt adhesive includes a specific styrene-butadiene-styrene block copolymer (hereinafter, referred to as "SBS"), as an essential component, that is polymerized in such a manner that a vinyl content rate is more than 35%, that is, the chemical structure of the 1,2-butadienyl form expressed by a chemical formula (1) below is contained in a ratio of more than 35% (e.g., see Japanese Laid-Open Patent Publication (Tokkai) No. 8-333563 (pages 2-3). However, this hotmelt adhesive has problems in that the viscosity at 120 °C is not sufficiently low, or the adhesion or the holding power to a paper diaper member is insufficient.

Chemical formula (1) : -CH₂-CH(CH=CH₂)-

### SUMMARY OF THE INVENTION

It is an object of the present invention to provide a method of obtaining a disposable product comprising applying a hotmelt adhesive in which at least one selected from the group consisting of the high degree of the adhesion and the holding power of the hotmelt adhesive to a paper diaper, the high softening point, the low viscosity, and the high heat stability is improved from the conventional hotmelt viscous composition. Furthermore, it is an object of the present invention to provide a method of obtaining a disposable product comprising applying a hotmelt adhesive having a comprehensively excellent performance.

The inventors of the present invention found as a result of in-depth research that a hotmelt adhesive including a predetermined ratio of a block copolymer including a specific amount of a specific styrene-butadiene-styrene block copolymer can be applied without being in contact even at a low temperature, and thus achieved the present invention.

According to one aspect, the present invention provides a novel method of obtaining a disposable product comprising applying a hotmelt adhesive comprising a component (I) : block copolymer, a component (II) : tackifier, and a component (III) : plasticizer,
wherein the component (I) : block copolymer comprises at least 30 wt% of styrene-butadiene-styrene block copolymer (A) (hereinafter, also referred to as "(A)") having a styrene content rate of 35 to 50 wt%, a styrene-butadiene diblock content rate of 50 to 80 wt%, and a viscosity of a 15 wt% toluene solution at 25°C of 20 to 40 mPa·s, and
the component (I) is contained in 17 to 30 wt% with respect to the total amount of the components (I), (II), and (III). This hotmelt adhesive can be preferably used for disposable products.

In one embodiment of the present invention, the component (I) comprises less than 70 wt% of an additional block copolymer (B) (hereinafter, also referred to as "(B)") other than (A), and
the additional block copolymer (B) is a compatible block copolymer.

In one embodiment of the present invention, the compatible block copolymer is at least one selected from the group consisting of a styrene-butadiene-styrene block copolymer other than (A), and a partially hydrogenated styrene-butadiene-styrene block copolymer in which a polymer block based on butadiene is partially hydrogenated.

Furthermore, in one embodiment of the present invention, the compatible block copolymer has a viscosity of a 15 wt% toluene solution at 25°C of 115 mPa·s or less.

In another embodiment of the present invention, the melt viscosity at 120°C is 10000 mPa·s or less, and the softening point is 75°C or more.

According to another aspect, the present invention provides a disposable product obtained by applying the above-described hotmelt adhesive at a temperature of 130°C or less in a non-contact manner.

In the present invention, the specific styrene-butadiene-styrene block copolymer (A) is contained in the specific amount in the component (I) : block copolymer, which is the base polymer of the hotmelt adhesive, and the component (I) is contained in the predetermined amount in the hotmelt adhesive, so that a hotmelt adhesive can be obtained in which at least one selected from the adhesion and the holding power of the hotmelt adhesive to the paper diaper member, the softening point, the viscosity and the heat stability is improved. Preferably, a hotmelt adhesive having an excellent balance of these properties can be obtained.

(A) has a specific chemical structure and property (that is, the styrene content rate is 35 to 50 wt%, the styrene-butadiene diblock content rate is 50 to 80 wt%, and the viscosity of a 15 wt% toluene solution at 25°C is 20 to 40 mPa·s), and preferably, a hotmelt adhesive having good balance of tackiness and holding power (cohesion) can be obtained. Furthermore, the viscosity of the hotmelt adhesive can be low even at a low temperature (e.g., 120°C), so that non-contact application can be easily performed at a low temperature.

### DESCRIPTION OF THE PREFERRED EMBODIMENTS

In the present invention, the "block copolymer" refers to a block copolymer comprising a polymer block based on at least one (preferably two or more) vinyl aromatic hydrocarbon (hereinafter, also referred to as "vinyl aromatic hydrocarbon block") and a polymer block based on at least one conjugated diene compound (hereinafter, also referred to as "conjugated diene compound block"), and includes a block copolymer in which an aliphatic double bond of a conjugated diene compound block is partially hydrogenated and a block copolymer in which an aliphatic double bond of a conjugated diene compound block is substantially completely hydrogenated.

The "vinyl aromatic hydrocarbon" refers to an aromatic hydrocarbon compound having vinyl group(s), and examples thereof include styrene, o-methylstyrene, p-methylstyrene, p-tert-butyl styrene, 1,3-dimethylstyrene, α-methylstyrene, vinylnaphthalene, and vinylanthracene. The vinyl aromatic hydrocarbon can be used alone or in combination. Styrene is particularly preferable as the vinyl aromatic hydrocarbon.

The "conjugated diene compound" refers to a diolefin compound having at least one pair of conjugated double bonds, and examples thereof include 1,3-butadiene, 2-methyl-1,3-butadiene (isoprene), 2,3-dimethyl-1,3-butadiene, 1,3-pentadiene, and 1,3-hexadiene. The conjugated diene compound can be used alone or in combination. As the conjugated diene compound, 1,3-butadiene is particularly preferable.

Therefore, in the present invention, the "component (I) : block copolymer" refers to a block copolymer as described above comprising at least 30 wt% of the styrene-butadiene-styrene block copolymer (A) having a styrene content rate of 35 to 50 wt%, a styrene-butadiene diblock content rate of 50 to 80 wt%, and a viscosity of a 15 wt% toluene solution at 25°C of 20 to 40 mPa.s.

In the present invention, the "styrene-butadiene-styrene block copolymer (A) " refers to a block copolymer having a structure of S-B-S comprising a polymer block based on styrene (hereinafter, also referred to as "styrene block (S)") and a polymer block based on butadiene (hereinafter, also referred to as "butadiene block (B)") in which the content rate of the styrene block (hereinafter, also referred to as "styrene content rate") is 35 to 50 wt%, the content rate of the diblock copolymer (hereinafter, also referred to as "diblock content rate" is 50 to 80 wt%, and the viscosity of a 15 wt% toluene solution at 25°C is 20 to 40 mPa.s.

The "styrene content rate" refers to the ratio of the styrene block contained in (A). The styrene content rate is preferably 35 to 50 wt%, more preferably 35 to 45 wt%, and particularly preferably 40 to 45 wt%. When the styrene content rate is less than 35 wt%, the holding power (cohesion) of the hotmelt adhesive itself can be reduced, and when the styrene content rate exceeds 50 wt%, the tackiness of the hotmelt adhesive itself can be reduced.

The "diblock content rate" refers to the ratio of the styrene-butadiene diblock copolymer (hereinafter, also referred to as "S-B") in (A). The diblock content rate is preferably 50 to 80 wt%, more preferably 55 to 75 wt%, and particularly preferably 60 to 70 wt%. When the diblock content rate is less than 50 wt%, the tackiness can be poor. In particular, when a load is applied in the shear direction at a high temperature, the hotmelt adhesive tends to be detached from the substrate of the diaper at its interface because of poor tackiness. As a result, the hotmelt adhesive tends to have a low holding power with respect to a polyolefin film such as a polyethylene film. When the diblock content rate exceeds 80 wt%, the holding power (cohesion) and the adhesion at a high temperature of the hotmelt adhesive can be reduced.

The "viscosity of a 15 wt% toluene solution at 25°C" refers to the viscosity of a solution having a concentration of 15 wt% using toluene as a solvent at 25°C, and can be measured with various viscometers such as a Brookfield BM viscometer (spindle No. 2). The viscosity of a 15 wt% toluene solution of (A) at 25°C is preferably 20 to 40 mPa·s, more preferably 20 to 35 mPa·s. When the viscosity of a 15 wt% toluene solution of (A) at 25°C is less than 20 mPa·s, the molecular weight of (A) is low. Therefore, the polymer is hardly solidified when producing (A), that is, the softening point can be low when it is formed into a hotmelt adhesive, and blocking can be caused in its production line or caused by cold flow after being molded. When the viscosity of a 15 wt% toluene solution at 25°C is more than 40 mPa·s, the viscosity of the hotmelt adhesive itself is high and can hardly be applied. In particular, non-contact application can be difficult at a low temperature.

It is preferable that (A) is polymerized so as to have a vinyl content rate of 35 wt% or less, that is, have the chemical structure of the 1,2-butadienyl form expressed by a chemical formula (1) below in a ratio of 35 wt% or less, and more preferably 20 wt% or less, and particularly preferably 15 wt% or less. When the vinyl content rate exceeds 35 wt%, the adhesion can be low, and the adhesion can be reduced over time.

Chemical formula (1) : -CH₂-CH (CH=CH₂) -

The component (I) comprises 30 wt% of (A) described above. The component (I) comprises preferably 50 wt% or more of (A), more preferably 60 wt% or more of (A), and particularly preferably 65 wt% or more of (A). When the content rate of (A) in the component (I) is less than 30 wt%, the hotmelt adhesive has a high viscosity, and the workability during application can be insufficient.

The component (I) is preferably 17 to 30 wt% with respect to the total amount of the components (I), (II), and (III), and more preferably 20 to 25 wt%. When the content rate of the component (I) is less than 17 wt%, the holding power (cohesion) of the hotmelt adhesive can be insufficient, and the softening point can be low. When the content rate of the component (I) is more than 30 wt%, the viscosity of the hotmelt adhesive can be high, and the workability during application can be poor.

Furthermore, when (A) contained in the component (I) contained in the hotmelt adhesive of the present invention has a melt flow rate (hereinafter, also referred to as "MFR") measured under the G condition of ASTM D 1238 of 60 to 140 g/10 min, it is satisfied that the viscosity of the 15 wt% toluene solution at 25°C is 20 to 40 mPa·s.

In this case, in the present invention, the "component (I) : block copolymer" refers to a block copolymer comprising at least 30 wt% of styrene-butadiene-styrene block copolymer (A) having a styrene content rate of 35 to 50 wt%, a styrene-butadiene diblock content rate of 50 to 80 wt%, and a melt flow rate measured under the G condition of ASTM D 1238 of 60 to 140 g/10 min.

Therefore, according to one aspect, the present invention provides a method of obtaining a disposable product comprising applying a hotmelt adhesive comprising a component (I) : block copolymer, a component (II) : tackifier, and a component (III) : plasticizer,
wherein the component (I) : block copolymer comprises at least 30 wt% of styrene-butadiene-styrene block copolymer (A) having a styrene content rate of 35 to 50 wt%, a styrene-butadiene diblock content rate of 50 to 80 wt%, and a melt flow rate measured under the G condition of ASTM D 1238 of 60 to 140 g/10 min, and
the component (I) is contained in 17 to 30 wt% with respect to the total amount of the components (I), (II), and (III).

The "block copolymer", the "styrene content rate" and the "styrene-butadiene diblock content rate" are those as described above.

In the present invention, the "melt flow rate (MFR)" refers to a value obtained by measurement under the G condition (200 °C, a load of 5.0 kg) of ASTM D 1238. The MFR is preferably 60 to 140 g/10 min, more preferably 70 to 120 g/10 min, and particularly preferably 80 to 100 g/10 min.

In the present invention, even if this specific (A) characterized by having an MFR of 60 to 140 g/10 min is contained in a specific amount in the component (I) : block copolymer that is the base polymer of the hotmelt adhesive, a hotmelt adhesive can be obtained in which at least one selected from the group consisting of the adhesion and the holding power of the hotmelt adhesive to the paper diaper member, the softening point, the viscosity, and the heat stability is improved. Preferably, a hotmelt adhesive having an excellent balance of these properties can be obtained.

Furthermore, in this case, since (A) has a specific chemical structure and property (that is, the styrene content rate is 35 to 50 wt%, the styrene-butadiene diblock content rate is 50 to 80 wt%, and the MFR is 60 to 140 g/10 min), preferably, a hotmelt adhesive having balanced tackiness and holding power (cohesion) can be obtained. Furthermore, the viscosity of the hotmelt adhesive can be low even at a low temperature (e.g., 120°C), so that non-contact application can be easily performed at a low temperature.

The hotmelt adhesive of the present invention may contain an additional block copolymers (B) other than (A) in a ratio of less than 70 wt%. In the specification of the present invention, "other than (A)" specifically means "not corresponding to the styrene-butadiene-styrene block copolymer having a styrene content rate of 35 to 50 wt%, a styrene-butadiene diblock content rate of 50 to 80 wt%, and a viscosity of a 15 wt% toluene solution at 25°C of 20 to 40 mPa.s". Therefore, "an additional block copolymer (B)" specifically refers to a block copolymer that does not correspond to the styrene-butadiene-styrene block copolymer having a styrene content rate of 35 to 50 wt%, a styrene-butadiene diblock content rate of 50 to 80 wt%, and a viscosity of a 15 wt% toluene solution at 25°C of 20 to 40 mPa·s. (B) is preferably a compatible block copolymer.

The "compatible block copolymer" refers to a block copolymer compatible with (A). Examples of such a compatible block copolymer include a styrene-butadiene-styrene block copolymer other than (A), and a partially hydrogenated styrene-butadiene-styrene block copolymer in which a polymer block based on butadiene is partially hydrogenated. Therefore, more specific examples of the compatible block copolymer include a styrene-butadiene-styrene block copolymer that does not correspond to the styrene-butadiene-styrene block copolymer having a styrene content rate of 35 to 50 wt%, a styrene-butadiene diblock content rate of 50 to 80 wt%, and a viscosity of a 15 wt% toluene solution at 25°C of 20 to 40 mPa·s, and a partially hydrogenated styrene-butadiene-styrene block copolymer in which a polymer block based on butadiene is partially hydrogenated. The styrene-butadiene-styrene block copolymer that does not corresponds to the styrene-butadiene-styrene block copolymer having a styrene content rate of 35 to 50 wt%, a styrene-butadiene diblock content rate of 50 to 80 wt%, and a viscosity of a 15 wt% toluene solution at 25°C of 20 to 40 mPa·s is preferable as the compatible block copolymer. The compatible block copolymer may contain SIS, as long as a desired hotmelt adhesive can be obtained.

The "partially hydrogenated styrene-butadiene-styrene block copolymer" is a block copolymer in which a block based on a conjugated diene compound is partially hydrogenated. The hydrogenation ratio of the block based on a conjugated diene compound is preferably 25 to 80%, more preferably 30 to 70%, and particularly preferably 30 to 50%.
Herein, the "hydrogenation ratio" of the block based on a conjugated diene compound refers to the ratio of double bonds that have been converted to saturated hydrocarbon bonds due to hydrogenation on the basis of all the aliphatic double bonds contained in the block based on the conjugated diene compound. The "hydrogenation ratio" is measured by an infrared spectrophotometer, a nuclear magnetic resonance device and the like.

Regarding the compatible block copolymer, the viscosity of a 15 wt% toluene solution at 25°C (hereinafter, also referred to as "the viscosity of the toluene solution" is preferably 115 mPa·s or less, more specifically more than 40 mPa·s and not more than 90 mPa·s, and particularly preferably more than 40 mPa·s and not more than 60 mPa·s. When the viscosity of a 15 wt% toluene solution at 25°C exceeds 115 mPa·s, the viscosity of the hotmelt adhesive itself can be high and it can be difficult to apply. In particular, for example, non-contact application can become difficult at a low temperature of 120°C or less. The viscosity of a 15 wt% toluene solution at 25°C can be measured in the manner as described above.

In the hotmelt adhesive of the present invention, the ratio (B)/((A) + (B)) of (B) with respect to the total amount of (A) and (B) is less than 0.7 (i.e., the ratio (A)/((A) + (B)) of (A) with respect to the total amount of (A) and (B) is 0.3 or more). The ratio (B)/((A) + (B)) may be less than 0.5, and may be less than 0.4. When the ratio (B)/((A) + (B) exceeds 0.7, the melt viscosity of the hotmelt adhesive can be high, and non-contact application can become difficult at a low temperature.

(A) and (B) can be produced by a known method by changing the reaction conditions as appropriate.
As (A), for example, a block copolymer named Asaprene JT96 (product name) manufactured by Asahi Chemical Industries Co. Ltd is available.

As (B), for example, Asaprene T436 (product name) manufactured by Asahi Chemical Industries Co. Ltd, TR2003 (product name) manufactured by JSR Corporation, TR2600 (product name) manufactured by JSR Corporation, stereon 857 (product name) manufactured by Firestone Inc., stereon 841A (product name) manufactured by Firestone Inc., Kraton D1118 (product name) manufactured by Kraton Polymers, TR2500 (product name) manufactured by JSR Corporation, Tufprene T125 (product name) manufactured by Asahi Chemical Industries Co. Ltd., or Tuftec (product name) manufactured by Asahi Chemical Industries Co. Ltd can be used.
(A) and (B) can be used alone or in combination.

In the present invention, there is no limitation regarding the component (II) : tackifier, as long as it is a tackifier commonly used in a hotmelt adhesive. Examples of the component (II) include natural rosin, modified rosin, hydrogenated rosin, glycerol ester of natural rosin, glycerol ester of modified rosin, pentaerythritol ester of natural rosin, pentaerythritol ester of modified rosin, pentaerythritol ester of hydrogenated rosin, a copolymer of natural terpene, a three dimensional polymer of natural terpene, hydrogenated derivatives of a copolymer of natural terpene, polyterpene resin, hydrogenated derivatives of phenol based modified terpene resin, aliphatic petroleum hydrocarbon resin, hydrogenated derivatives of aliphatic petroleum hydrocarbon resin, aromatic petroleum hydrocarbon resin, hydrogenated derivatives of aromatic petroleum hydrocarbon resin, cyclic aliphatic petroleum hydrocarbon resin, and hydrogenated derivatives of cyclic aliphatic petroleum hydrocarbon resin. The tackifier can be used alone or in combination.

The tackifier can be in the form of a liquid, as long as the color is from colorless to pale yellow, it is substantially odorless, and the heat stability is good. In view of these characteristics overall, hydrogenated derivatives of resins or the like are preferable as the tackifier.

When the hotmelt adhesive of the present invention is used for disposable products such as sanitary napkins or paper diapers, it is preferable that the tackifier is colorless or white. In the present invention, "odorless" means that there is almost no odor, as shown in the self control of the JAPAN HYGIENE PRODUCTS INDUSTRY ASSOCIATION.

The mixing ratio of the component (II) is preferably 45 to 80 wt%, and more preferably 50 to 75 wt%, with respect to the total of the components (I) to (III). When the mixing ratio of the component (II) is less than 45 wt%, the adhesion of the hotmelt adhesive can be low, and when the mixing ratio of the component (II) is more than 80 wt%, the hotmelt adhesive can become fragile and the low temperature adhesion can be deteriorated.

As the component (II), a commercially available product can be used. Examples of such a component (II) include ECR5600 (product name) manufactured by Tonex Co., Ltd., Marukaclear H (product name) manufactured by Maruzen Petrochemical Co., Ltd., Clearon K100 (product name) manufactured by Yasuhara Chemical Co., Ltd., ARKON M100 (product name) manufactured by Arakawa Chemical Industries, Ltd., Imarv S100 (product name) manufactured by Idemitsu Petrochemical Co., Ltd., Clearon K4090 (product name) manufactured by Yasuhara Chemical Co., Ltd., ECR231 C (product name) manufactured by Tonex Co., Ltd., and REGALITE R7100 (product name) manufactured by Eastman Chemical Company. The component (II) can be used alone or in combination.

In the present invention, the "component (III) : plasticizer" is generally a plasticizer that is mixed for the purpose for reducing the melt viscosity, providing the flexibility, adjusting the adhesion of a hotmelt adhesive, and any plasticizer can be used, as long as it is compatible with the component (I). For example, paraffin based oil, naphthene based oil, and aromatic oil can be used as the component (III). Paraffin based oil that is colorless and is substantially odorless is particularly preferable.

The mixing ratio of the component (III) is preferably 3 to 25 wt%, and more preferably 5 to 20 wt%, with respect to the total of the components (I) to (III). When the mixing ratio of the component (III) is less than 3 wt%, the flexibility of the hotmelt adhesive can be low, and when the mixing ratio of the component (III) is more than 25 wt%, the adhesive property of the hotmelt adhesive can be deteriorated.

As the component (III), a commercially available product can be used. Examples of such a component (III) include LP350 (product name) manufactured by Kukdon Oil & Chem, Diana Fresia S32 (product name) manufactured by Idemitsu Kosan Co., Ltd., Diana Process Oil PW-90 (product name) manufactured by Idemitsu Kosan Co., Ltd., EnerperM1930 (product name) manufactured by BP Chemicals, Kaydol (product name) manufactured by Crompton Corporation, Whiterex 335 (product name) manufactured by Tonen General Sekiyu K. K., and Primo1352 (product name) manufactured by Esso.
The component (III) can be used alone or in combination.

The hotmelt adhesive of the present invention may contain a component (IV) : stabilizing agent, in addition to the components (I) to (III). There is no limitation regarding the "component (IV) : a stabilizing agent", as long as it is mixed for the purpose for preventing a decrease in the molecular weight, gelatinization, coloring, odor occurrence or the like due to the heat of the hotmelt adhesive, and improving the stability of the hotmelt adhesive. Examples of the stabilizing agent include an antioxidant and an ultraviolet absorber. The ultraviolet absorber is used to improve the light resistance of the hotmelt adhesive. The antioxidant is used to prevent oxidation deterioration of the hotmelt adhesive. There is no limitation regarding the antioxidant and the ultraviolet absorber, as long as they generally can be used for hygiene materials. Examples of antioxidants include a phenol based antioxidant, a sulfur based antioxidant, and a phosphorus based antioxidant. Examples of ultraviolet absorbers include a benzotriazole based ultraviolet absorber and a benzophenon based ultraviolet absorber. Furthermore, a lactone based stabilizing agent can be added. They can be used alone or in combination.

The mixing ratio of the component (IV) is preferably 0.1 to 4 parts by weight, and particularly preferably 0.3 to 2 parts by weight, with respect to 100 parts by weight of the components (I) to (III). When the mixing ratio of the component (IV) is less than 0.1 parts by weight, a decrease in the molecular weight, gelatinization, coloring, odor occurrence or the like due to heat may not be prevented. When the mixing ratio of the component (IV) is more than 4 parts by weight, the effect commensurate with the added amount cannot be obtained, which is also disadvantageous in the cost.

As the component (IV), a commercially available product can be used. Examples of such a component (IV) include Sumilizer GM (product name) manufactured by Sumitomo Chemical Co., Ltd., Sumilizer TPD (product name) manufactured by Sumitomo Chemical Co., Ltd., Irganox 1010 (product name) manufactured by Chiba Speciality Chemicals, JF77 (product name) manufactured by Johoku Chemical Co., Ltd, Irganox HP2225FF (product name) manufactured by Chiba Speciality Chemicals, Irgafos 168 (product name) manufactured by Chiba Speciality Chemicals, Irganox 1520 (product name) manufactured by Chiba Speciality Chemicals, and Sumilizer TPS (product name) manufactured by Sumitomo Chemical Co., Ltd. The component (IV) can be used alone or in combination.

The hotmelt adhesive of the present invention may contain a component (V) : a particulate filler, in addition to the component (IV). There is no limitation regarding the "component (V): stabilizing agent", as long as it is commonly used. Examples of the particulate filler include mica, calcium carbonate, kaoline, talc, titanium oxide, kieselguhr, urea based resin, styrene beads, fired clay, and starch. They are preferably spherical, and there is no limitation regarding the size (diameter in the case of spherical shape).

The hotmelt adhesive of the present invention can be produced by mixing the components (I) to (V) in a predetermined ratio, if necessary, mixing various additives, and heating and melting the components for blending. More specifically, the components are fed to a melt blending pot provided with a stirrer and are heated for mixture.

It is preferable that the obtained hotmelt adhesive has a melt viscosity at 120°C of 10000 mPa·s or less, and a softening point of 75°C or more. The "melt viscosity" refers to the viscosity of the hotmelt adhesive in the melt form. This can be measured with a Brookfield RVT type viscometer (spindle No. 27). The "softening point" is a temperature at which the hotmelt adhesive that has been heated becomes soft and begins to be deformed. This can be measured by the ring & ball method (defined by the Japan Adhesive Industry Association Standard JAI-7-1999).

In the case where the melt viscosity at 120°C of the hotmelt adhesive of the present invention is 10000 mPa·s or less and the softening point is 75°C or more, the hotmelt adhesive can be supplied in the form of a liquid or semi-liquid. The hotmelt adhesive is stored in an insulated container in the form of a liquid or semi-liquid, and can be supplied as it is. Therefore, the hotmelt adhesive is environmentally very preferable. In other words, when producing a hotmelt adhesive, the generation of waste material can be reduced and power consumption can be saved.

Furthermore, the holding power of the hotmelt adhesive of the present invention that is evaluated by the method described in the example is preferably 10 min or more, more preferably 30 min or more, and particularly preferably 60 min or more.

Furthermore, the peeling strength of the hotmelt adhesive of the present invention that is evaluated by the method described in the example is preferably 900 gf/inch (8.8 N/2.54 cm) or more, more preferably 1000 gf/inch (9.8 N/2.54 cm) or more.

Furthermore, the loop tack of the hotmelt adhesive of the present invention that is evaluated by the method described in the example is preferably 1000 gf/inch (9.8 N/2.54 cm) or more, more preferably 1500 gf/inch (14.7 N/2.54 cm) or more, and particularly preferably 2000 gf/inch (19.6 N/2.54 cm) or more.

Furthermore, in the hotmelt adhesive of the present invention, it is preferable that odor does not occur, according to a method for evaluating heat stability described in the example. The viscosity maintaining ratio of the hotmelt adhesive of the present invention evaluated by the method for evaluating heat stability described in the example is preferably 85% or more, more preferably 90% or more, and particularly preferably 95% or more.

The hotmelt adhesive of the present invention can be used primarily for disposable products. The "disposable products" can be any product, as long as they are, for example, so-called hygiene materials. Specific examples of hygiene materials include sanitary napkins, pet sheets, hospital gowns, and sanitary gowns for operation.

According to another aspect, the present invention provides a disposable product obtained by non-contact application of the hotmelt adhesive at 130°C or less. The disposable product is produced by attaching at least one selected from the group consisting of woven fabric, non-woven fabric, rubber, resin and paper and a polyolefin film with the hotmelt adhesive of the present invention. As the polyolefin film, a polyethylene film is preferable in terms of durability, cost and the like.

In the production line of disposable products, in general, the hotmelt adhesive is applied on at least one of a member (e.g., non-woven fabric) of a disposable product and a polyolefin film, and the film and the member are attached by pressure, and thus a disposable product is produced. The hotmelt adhesive can be sprayed from various spraying devices for application. In the present invention, "non-contact application" refers to a method in which the hotmelt adhesive is applied without bringing the spraying device in contact with the member or the film. Specific examples of the non-contact application method include spiral coating by which application is performed spirally, omega coating by which application is performed sinuously, control seam coating, slot spray coating or curtain spray coating by which application is performed planarly, and dot coating by which application is performed in dots.

### Examples

Hereinafter, the present invention will be described more specifically and in detail by way of examples and comparative examples, but these examples are only embodiments of the present invention and the present invention is not limited thereby.
In the description of the examples, the portion in which the solvent is not considered is used as the basis of parts by weight and % by weight, unless otherwise specified.

The components (I) to (IV) used in the examples are shown below.
As (A) contained in the component (I), (A1) Asaprene JT96 (product name) manufactured by Asahi Chemical Industries Co., Ltd. (styrene-butadiene-styrene block copolymer, the viscosity of a 15 wt% toluene solution at 25°C : 28 mPa·s, styrene content rate :43 wt%, diblock content rate : 63 wt%, the content rate of the form of 1,2-butadienyl : about 14 wt%, the melt flow rate : about 100 g/10 min) was used.

The "content rate of the form of 1,2-butadienyl" refers to the ratio of the chemical structure expressed by a chemical formula (1) below that is contained in the component (A).

Chemical formula (1) : -CH₂-CH(CH=CH₂)-

As (B), the following block copolymer was used.
(B1) Asaprene T436 (product name) manufactured by Asahi Chemical Industries Co., Ltd. (styrene-butadiene-styrene block copolymer, the viscosity of a 15 wt% toluene solution at 25°C: 110 mPa·s, styrene content rate :30 wt%, diblock content rate : 50 wt%, the content rate of the form of 1,2-butadienyl : about 14 wt%, MFR : 6 g/10 min);
(B2) TR2003 (product name) manufactured by Japan synthetic rubber Co. Ltd. (styrene-butadiene-styrene block copolymer, the viscosity of a 15 wt% toluene solution at 25°C :75 mPa·s, styrene content rate :43 wt%, diblock content rate : 10 wt%, the content rate of the form of 1,2-butadienyl : about 20 wt%, MFR : 18 g/10 min);
(B3) TR2600 (product name) manufactured by Japan synthetic rubber Co. Ltd. (styrene-butadiene-styrene block copolymer, the viscosity of a 15 wt% toluene solution at 25°C:58 mPa·s, styrene content rate :32 wt%, diblock content rate : 70 wt%, the content rate of the form of 1,2-butadienyl : about 13 wt%, MFR : 40 g/10 min);
(B4) stereon 857 (product name) manufactured by Firestone Inc. (styrene-butadiene-styrene block copolymer, the viscosity of a 15 wt% toluene solution at 25°C:48 mPa·s, styrene content rate : 44 wt%, diblock content rate : about 10 wt% or less, the content rate of the form of 1,2-butadienyl : about 57 wt%, MFR : 13 g/10 min);
(B5) stereon 841A (product name) manufactured by Firestone Inc. (styrene-butadiene-styrene block copolymer, the viscosity of a 15 wt% toluene solution at 25°C:85 mPa·s, styrene content rate : 44 wt%, diblock content rate : 0 wt%, the content rate of the form of 1,2-butadienyl : about 13 wt%, MFR : 12 g/10 min);
(B6) Kraton D1118 (product name) manufactured by Kraton Polymers (styrene-butadiene-styrene block copolymer, the viscosity of a 15 wt% toluene solution at 25°C : 75 mPa·s, styrene content rate : 31 wt%, diblock content rate : 80 wt%, the content rate of the form of 1,2-butadienyl : about 13 wt%, MFR : 10 g/10 min); and
(B7) Vector 4411 D (product name) manufactured by DEXCO (styrene-isoprene-styrene block copolymer, the viscosity of a 15 wt% toluene solution at 25°C : 30 mPa.s, styrene content rate : 44 wt%, diblock content rate : 0 wt% : MFR : 40 g/10 min).

As the component (II), (C1) ECR5600 (product name) manufactured by Tonex Co., Ltd (petroleum based tackifier resin), (C2) Mulkaclear H (product name) manufactured by Maruzen Petrochemical Co., Ltd. (petroleum based liquid tackifier resin), and (C3) Clearon K100 (product name) manufactured by Yasuhara Chemical Co., Ltd. (hydrogenated terpene resin) were used.

As the component (III), (D1) LP350 (product name) manufactured by Kukdon Oil & Chem (paraffin oil) and (D2) Diana Fresia S32 (product name) manufactured by Idemitsu Kosan Co., Ltd. (paraffin oil) were used.

As the component (IV), (E1) Sumilizer GM (product name) manufactured by Sumitomo Chemical Co., Ltd. (phenol based primary antioxidant), (E2) Sumilizer TPD (product name) manufactured by Sumitomo Chemical Co., Ltd. (sulfur based secondary antioxidant), (E3) Irganox 1010 (product name) manufactured by Chiba Speciality Chemicals (phenol based primary antioxidant) and (E4) JF77 (product name) manufactured by Johoku Chemical Co., Ltd (ultraviolet absorber) were used.

The viscosity of a 15 wt% toluene solution at 25°C of (A) and (B) was measured with a Brookfield BM viscometer (spindle No. 2) at 25°C. The melt flow rate was measured under the G condition of ASTM D 1238.

Production of hotmelt adhesives of Examples 1 to 8 and Comparative Examples 1 to 8
The components shown in Tables 1 and 2 below were mixed at the ratios shown in these tables, and melted and mixed at about 150 °C to produce the hotmelt adhesives of Examples 1 to 8 and Comparative Examples 1 to 8.

**Table 1**

| Composition ^{a)} | Example | | | | | | | |
|---|---|---|---|---|---|---|---|---|
| | 1 | 2 | 3 | 4 | 5 | 6 | 7 | 8 |
| Component (I) | | | | | | | | |
| (A1) | 23 | 25 | 25 | 7 | 13 | 15 | 17 | 30 |
| (B1) | | | | 15 | | | | |
| (B2) | | | | | | 9 | | |
| (B3) | | | | | 10 | | | |
| (A)+(B) | 23 | 25 | 25 | 22 | 23 | 24 | 17 | 30 |
| (A)/((A)+(B)) | 1 | 1 | 1 | 0.31 | 0.56 | 0.63 | 1 | 1 |
| Component (II) | | | | | | | | |
| (C1) | 59 | 50 | 55 | 56 | 30 | 58 | 53 | 50 |
| (C2) | | 20 | | | | | 25 | |
| (C3) | | | | | 29 | | | |
| Component (III) | | | | | | | | |
| (D1) | | | 20 | | | | | |
| (D2) | 18 | 5 | | 22 | 18 | 18 | 5 | 20 |
| Component (IV) | | | | | | | | |
| (E1) | 0.2 | 0.3 | 0.3 | 0.3 | 0.2 | 0.2 | 0.3 | 0.3 |
| (E2) | 0.3 | 0.3 | 0.3 | 0.3 | 0.3 | 0.3 | 0.3 | 0.3 |
| (E3) | | | | | | | | |
| (E4) | 0.3 | 0.3 | 0.3 | 0.3 | | | 0.3 | 0.3 |

| | | | | | | | | |
|---|---|---|---|---|---|---|---|---|
| a) The unit of each component is parts by weight. | | | | | | | | |

**Table 2**

| Composition ^{a)} | Comparative Example | | | | | | | |
|---|---|---|---|---|---|---|---|---|
| | 1 | 2 | 3 | 4 | 5 | 6 | 7 | 8 |
| Component (I) | | | | | | | | |
| (A1) | 15 | 33 | | | | | | |
| (B4) | | | 22 | | | | | |
| (B5) | | | | 22 | 25 | | 11 | |
| (B6) | | | | | | 25 | | |
| (B7) | | | | | | | 11 | 25 |
| (A)+(B) | 15 | 33 | 22 | 22 | 25 | 25 | 22 | 25 |
| (A)/((A)+(B)) | 1 | 1 | 0 | 0 | 0 | 0 | 0 | 0 |
| Component (II) | | | | | | | | |
| (C1) | 55 | 42 | 54.5 | 54.5 | 55 | 55 | 57.5 | 55 |
| (C2) | 25 | | | | | | 25 | |
| Component (III) | | | | | | | | |
| (D1) | | | 23.5 | 23.5 | 20 | 20 | 20 | 20 |
| (D2) | 5 | 25 | | | | | | |
| Component (IV) | | | | | | | | |
| (E1) | 0.3 | 0.3 | 0.3 | 0.3 | 0.3 | 0.3 | | |
| (E2) | 0.3 | 0.3 | 0.3 | 0.3 | 0.3 | 0.3 | | |
| (E3) | 0.3 | 0.3 | 0.3 | 0.3 | 0.3 | 0.3 | 0.3 | 0.3 |
| (E4) | | | | | | | 0.5 | 1.0 |

| | | | | | | | | |
|---|---|---|---|---|---|---|---|---|
| a) The unit of each component is parts by weight. | | | | | | | | |

Regarding the thus obtained hotmelt adhesives of Examples 1 to 8 and Comparative Examples 1 to 6, the characteristics of the softening point, the melt viscosity, the holding power, the peeling strength, the loop tack, and the heat stability were investigated. The results were shown in Tables 3 and 4. The characteristics were investigated in the following manner.

### Softening point

The softening point of the hotmelt adhesive was measured by the ring & ball method (defined by the Japan Adhesive Industry Association Standard JAI-7-1999). The softening point of the hotmelt adhesive of Example 1 was 79.5°C.

### Melt viscosity

Regarding the melt viscosity, a hotmelt adhesive was heated and melted, and the viscosity of the melt adhesive was measured at 120°C and 140°C with a Brookfield RVT type viscometer (spindle No. 27). The viscosity of the hotmelt adhesive of Example 1 at 120°C was 4500 mPa·s, and the viscosity at 140°C was 1700 mPa·s.

### Holding power

A hotmelt adhesive was applied onto a PET film having a thickness of 50 µm such that the thickness was 50 µm. This PET film was shaped with a width of 2.5 cm and was used as a test sample. A polyethylene film having a thickness of 100 µm was attached to this test sample at 20°C such that the attached area was 1.0 cm × 2.5 cm, and then a weight of 1 kg was applied in the direction perpendicular to the attached surface, and was stored at 40°C. Then the time until the weight of 1 kg was dropped was measured, and this was used as holding power. The holding power of the hotmelt adhesive of Example 1 was 67 min.

### Peeling strength

A hotmelt adhesive was applied onto a PET film having a thickness of 50 µm such that the thickness was 50µm. This PET film was shaped with a width of 2.5 cm and was used as a test sample. A polyethylene film having a thickness of 100 µm was attached to this test sample at 20°C, and was stored at 20°C for one day. Thereafter, peeling was performed at a pulling rate of 300 mm/min at 40 °C, and the peeling strength was measured. The peeling of the hotmelt adhesive of Example 1 was 1230 gf/inch (12.1 N/2.54 cm).

### Loop tack

A hotmelt adhesive was applied onto a PET film having a thickness of 50 µm such that the thickness was 50 µm. This PET film was shaped with a size of 2.5 cm × 10 cm and was used as a test sample. This test sample was wound in a loop such that the adhesive surface (surface on which an adhesive was applied) was outside, and was brought in contact with a PE plate at 20°C at a rate of 300 mm/min. Thereafter, the test sample was peeled from the PE plate at a rate of 300 mm/min, and the peeling strength at that time was measured, and this was taken as the initial loop tack. On the other hand, the above-described test sample was stored at 20°C for one week, and then was brought in contact with a PE plate at 20°C at a rate of 300 mm/min. Thereafter, the test sample was peeled from the PE plate at a rate of 300 mm/min, and the peeling strength at that time was measured, and this was taken as the loop tack after one week. The initial loop tack of the hotmelt adhesive of Example 1 was 3100 gf/inch (30.4 N/2.54 cm), and the loop tack after one week was 3700 gf/inch (36.3 N/2.54 cm).

### Heat stability

The hotmelt adhesive in an amount of 30 g was placed in a glass bottle, and was stored in a hot air circulated dryer at 120 °C for one week. Thereafter, the melt viscosity was measured by the above-described method, and the viscosity change rate (%) after storage with respect to the viscosity of the initial stage (before the treatment with the dryer) was calculated. Similarly, the hotmelt adhesive in an amount of 30 g was placed in a glass bottle, and was stored in a hot air circulated dryer at 120 °C for one week. Thereafter, odor was evaluated qualitatively by a sensory test. Then, the hotmelt adhesive after storage was compared with that in the initial stage, and regarding odor, the hotmelt adhesive that generates odor was denoted by "A", the hotmelt adhesive that does not generate odor was denoted by "C". In the hotmelt adhesive adhesion of Example 1, odor was not generated. The viscosity change rate (%) was 96%.

**Table 3**

| Characteristics | Example | | | | | | | |
|---|---|---|---|---|---|---|---|---|
| | 1 | 2 | 3 | 4 | 5 | 6 | 7 | 8 |
| softening point ^{a)} | 79.5 | 85 | 86.6 | 82.8 | 81 | 81 | 76 | 89.2 |
| melt viscosity ^{b)} | | | | | | | | |
| 120°C | 4500 | 8100 | 6700 | 9300 | 7700 | 9300 | 2700 | 10000 |
| 140°C | 1700 | 2800 | 2300 | 3800 | 2600 | 3700 | 1000 | 3580 |
| holding power ^{c)} | 67 | 320 | 27 | 12 | 30 | 60 | 22 | 11 |
| peeling strength ^{d)} | 1230 | 1300 | 1210 | 1200 | 1130 | 1300 | 1000 | 1210 |
| | 12.1 | 12.7 | 11.9 | 11.8 | 11.0 | 12.7 | 9.8 | 11.9 |
| loop tack ^{e)} | | | | | | | | |
| initial stage | 3100 | 1700 | 2400 | 2100 | 2800 | 1700 | 1000 | 1700 |
| | 30.4 | 16.7 | 23.5 | 20.6 | 27.4 | 16.7 | 9.8 | 16.7 |
| after one week | 3700 | 2400 | 2800 | 2300 | 2800 | 1600 | 1050 | 2100 |
| | 36.3 | 23.5 | 27.4 | 22.5 | 27.4 | 15.7 | 10.3 | 20.6 |
| heat stability ^{f)} | | | | | | | | |
| Odor | A | A | A | A | A | A | A | A |
| viscosity maintaining ratio | 96 | 94 | 95 | 92 | 95 | 93 | 96 | 94 |

| | | | | | | | | |
|---|---|---|---|---|---|---|---|---|
| a) The unit of the softening point was °C. b) The unit of the melt viscosity is mPa·s. c) The holding power was measured at 40 °C and the unit thereof is minutes. d) The peeling strength was measured at 40 °C and the unit thereof is gf/inch in the upper fields, and N/2.54 cm in the lower fields. e) The loop tack was measured at 20 °C and the unit thereof is gf/inch in the upper fields, and N/2.54 cm in the lower fields. f) The heat stability is evaluated with one week storage at 120°C, and the unit of the viscosity maintaining ratio is %. | | | | | | | | |

**Table 4**

| characteristics | Comparative Example | | | | | | | |
|---|---|---|---|---|---|---|---|---|
| | 1 | 2 | 3 | 4 | 5 | 6 | 7 | 8 |
| softening point ^{a)} | 72.6 | 93.6 | 89.0 | 81.0 | 85.6 | 94.0 | 83.0 | 95.4 |
| melt viscosity ^{b)} | | | | | | | | |
| 120°C | 2125 | 12000 | 12300 | 12500 | 21550 | 21800 | 12000 | 16500 |
| 140°C | 825 | 3810 | 3190 | 4630 | 8150 | 7130 | 4000 | 4000 |
| holding power ^{c)} | 20 | 1 | 9 | 6 | 14 | 35 | 20 | 11 |
| peeling strength ^{d)} | 1220 | 260 | 1430 | 1400 | 1600 | 830 | 1600 | 800 |
| | 12.0 | 2.5 | 14.0 | 13.7 | 15.7 | 8.1 | 15.7 | 7.8 |
| loop tack ^{e)} | | | | | | | | |
| initial stage | 1100 | 950 | 2300 | 2500 | 2400 | 3100 | 1800 | 1400 |
| | 10.8 | 9.3 | 22.5 | 24.5 | 23.5 | 30.4 | 17.6 | 13.7 |
| after one week | 1700 | 980 | 560 | 2200 | 2200 | 3100 | 2500 | 1100 |
| | 16.7 | 9.6 | 5.5 | 21.6 | 21.6 | 30.4 | 24.5 | 10.8 |
| heat stability ^{f)} | | | | | | | | |
| odor | A | A | A | A | A | A | B | C |
| viscosity maintaining ratio | 96 | 91 | 94 | 93 | 91 | 90 | 73 | 70 |

| | | | | | | | | |
|---|---|---|---|---|---|---|---|---|
| a) The unit of the softening point was °C. b) The unit of the melt viscosity is mPa·s. c) The holding power was measured at 40 °C and the unit thereof is minutes. d) The peeling strength was measured at 40 °C and the unit thereof is gf/inch in the upper fields, and N/2.54 cm in the lower fields. e) The loop tack was measured at 20 °C and the unit thereof is gf/inch in the upper fields, and N/2.54 cm in the lower fields. f) The heat stability is evaluated with one week storage at 120°C, and the unit of the viscosity maintaining ratio is %. | | | | | | | | |

As shown in Table 3, the hotmelt adhesives of Examples 1 to 8 exhibit a low melt viscosity even at 120°C while keeping the softening point high, and are excellent in the application property at low temperatures. Furthermore, the holding power, the peeling strength, and the adhesion and the retention of the tackiness are appropriate and their balance is sufficient.
Furthermore, the heat stability is good.

On the other hand, as shown in Table 4, the hotmelt adhesive of Comparative Example 1 has a low softening point. The hotmelt adhesives of Comparative Examples 2 to 4 have a high melt viscosity at 120°C and a small holding power. The hotmelt adhesives of Comparative Examples 5 and 6 have a high melt viscosity at 120°C. The hotmelt adhesives of Comparative Examples 7 and 8 have a high melt viscosity at 120°C, and poor heat stability.

The hotmelt adhesive of the present invention contains component (I) containing the specific amount of the specific (A), and the components (II) and (III), and contains the predetermined amount of the component (I) with respect to the total amount of the components (I) to (III). Therefore, a hotmelt adhesive can be provided in which at least one selected from the group consisting of the degree of the adhesion and the holding power to a paper diaper, the high softening point, the low viscosity, and the high heat stability is improved. Preferably, a hotmelt adhesive having a low viscosity at 120 °C while keeping the softening point high and having excellent balance of the adhesion and holding power can be provided.

The conventional hotmelt adhesive was heated to a high temperature such as 130 °C or more to reduce its viscosity for the purpose of improving the application workability and then was applied. However, the hotmelt adhesive of the present invention can have a comparatively low viscosity even at low temperatures such as 120°C, and can be applied at 120°C. Therefore, since application is possible at 120°C, the problem that a polyolefin film constituting paper diaper is dissolved or wrinkled can be avoided by applying the hotmelt adhesive when producing paper diaper.

The hotmelt adhesive of the present invention can be stored in an insulated container in the form of a liquid or a semi-liquid, and therefore is environmentally and hygienically preferable. Furthermore, the hotmelt adhesive of the present invention that does not contain SIS is more preferable because thermal instability due to the SIS can be avoided.

The hotmelt adhesive of the present invention preferably has a softening point of 75°C or more, and has a melt viscosity of 10000 mPa·s or less at 120°C. Therefore, even if disposable products produced with the hotmelt adhesive of the present invention are stored in a warehouse in summer, the problem that the member and the film are peeled apart is alleviated, preferably prevented. Furthermore, the hotmelt adhesive of the present invention has excellent balance of the adhesion and the holding power (cohesion), so that disposable products can be used with ease and stored for a long time.

## Claims

1. A method of obtaining a disposable product comprising applying in a non-contact manner on at least one of a member of a disposable product and a polyolefin film a hotmelt adhesive at a temperature of 130°C or less and attaching the member to the film using pressure, **characterized in** said adhesive comprises a block copolymer component (I), a tackifier component (II) and a plasticizer component (III), component (I) present in amounts of 17 to 30 wt% with respect to the total amount of components (I), (II), and (III), and comprising at least 30 wt% of styrene-butadiene-styrene block copolymer (A) having a styrene content of 35 to 50 wt%, a styrene-butadiene diblock content of 50 to 80 wt%, and a viscosity in a 15 wt% toluene solution at 25°C of 20 to 40 mPas.

2. The method of claim 1 wherein component (I) comprises less than 70 wt% of an additional compatible block copolymer (B) selected from the group consisting of a styrene-butadiene-styrene block copolymer other than (A), and a partially hydrogenated styrene-butadiene-styrene block copolymer in which a polymer block based on butadiene is partially hydrogenated.

3. The method of claim 1 wherein the adhesive has a melt viscosity at 120°C of 10000 mPas or less, and a softening point of 75°C or more.

4. The method of claim 1 wherein the adhesive is in a form of a liquid or a semi-liquid.

5. The method of claim 1 wherein said film is a polyethylene film.

6. The adhesive of claim 5 wherein the adhesive is applied to said film.

7. The method of claim 1 wherein said member is a woven fabric, a nonwoven fabric or paper.

8. A disposable product obtained by applying in a non-contact manner on at least one of a member of a disposable product and a polyolefin film a hotmelt adhesive at a temperature of 130°C or less and attaching the member to the film using pressure, **characterized in** said adhesive comprises a block copolymer component (I), a tackifier component (II) and a plasticizer component (III), component (I) present in amounts of 17 to 30 wt% with respect to the total amount of components (I), (II), and (III), and comprising at least 30 wt% of styrene-butadiene-styrene block copolymer (A) having a styrene content of 35 to 50 wt%, a styrene-butadiene diblock content of 50 to 80 wt%, and a viscosity in a 15 wt% toluene solution at 25°C of 20 to 40 mPas.

## Patentansprüche

1. Verfahren zur Herstellung eines Einwegprodukts umfassend die Anwendung eines Schmelzklebstoffs in berührungsfreier Weise auf zumindest einen Vertreter eines Einwegprodukts und einen Polyolefinfilm bei einer Temperatur von 130 °C oder weniger und Befestigung des Vertreters am Film unter Aufwendung von Druck, **dadurch** charakterisiert, dass der Klebstoff eine Blockcopolymer Komponente (I), eine Klebrigmacherkomponente (II) und eine Weichmacherkomponente (III) umfasst, wobei die Komponente (I) in Mengen von 17 bis 30 Gew.% bezogen auf die Gesamtmengen der Komponenten (I), (II) und (III) vorhanden ist, und umfassend zumindest 30 Gew.% Styrol-Butadien-Styrol Blockcopolymer (A), das einen Styrol Gehalt von 35 bis 50 Gew.%, einen Styrol-Butadien Biblockgehalt von 50 bis 80 Gew.% und eine Viskosität von 20 bis 40 mPas bei 25 °C in 15 Gew.%iger Toluol-Lösung hat.

2. Verfahren nach Anspruch 1, wobei die Komponente (I) weniger als 70 Gew.% eines weiteren kompatiblen Blockcopolymers (B) umfasst, dass aus der Gruppe bestehend aus Styrol-Butadien-Styrol Blockcopolymeren, die anders sind als (A), und teilweise hydrierten Styrol-Butadien-Styrol Blockcopolymeren, in denen ein auf Butadien basierender Polymerblock teilweise hydriert ist, ausgewählt ist.

3. Verfahren nach Anspruch 1, wobei der Klebstoff bei 120 °C eine Schmelzviskosität von 10000 mPas oder weniger und einen Erweichungspunkt von 75 °C oder mehr hat.

4. Verfahren nach Anspruch 1, wobei der Klebstoff in Form einer Flüssigkeit oder semi-Flüssigkeit ist.

5. Verfahren nach Anspruch 1, wobei besagter Film ein Polyethylen Film ist.

6. Verfahren nach Anspruch 5, wobei der Klebstoff auf besagten Film angewendet wird.

7. Verfahren nach Anspruch 1, wobei der Vertreter ein Gewebe, ein Vliesstoff oder ein Papier ist.

8. Einwegprodukt, dass durch Anwendung eines Schmelzklebstoffs in berührungsfreier Weise auf zumindest einen Vertreter eines Einwegprodukts und einen Polyolefinfilm bei einer Temperatur von 130 °C oder weniger und Befestigung des Vertreters am Film unter Aufwendung von Druck erhalten wird, **dadurch** charakterisiert, dass der Klebstoff eine Blockcopolymer Komponente (I), eine Klebrigmacherkomponente (II) und eine Weichmacherkomponente (III) umfasst, wobei die Komponente (I) in Mengen von 17 bis 30 Gew.% bezogen auf die Gesamtmengen der Komponenten (I), (II) und (III) vorhanden ist, und umfassend zumindest 30 Gew.% Styrol-Butadien-Styrol Blockcopolymer (A) das einen Styrol Gehalt von 35 bis 50 Gew.%, einen Styrol-Butadien Biblockgehalt von 50 bis 80 Gew.% und eine Viskosität von 20 bis 40 mPas bei 25 °C in 15 Gew.%iger Toluol-Lösung hat.

## Revendications

1. Procédé d'obtention d'un produit à jeter après usage comprenant les étapes consistant à appliquer sans contact, sur au moins l'un d'un élément d'un produit à jeter après usage et d'un film polyoléfinique, un adhésif thermofusible à une température de 130°C ou moins et à attacher l'élément au film par utilisation de pression, **caractérisé en ce que** ledit adhésif comprend un composant (I) qui est un copolymère à blocs, un composant (II) qui est un agent collant et un composant (III) qui est un plastifiant, le composant (I) étant présent en des quantités de 17 à 30 % en poids par rapport à la quantité totale des composants (I), (II) et (III), et comprenant au moins 30 % en poids de copolymère à blocs styrène-butadiène-styrène (A) ayant une teneur en styrène de 35 à 50 % en poids, une teneur en diblocs styrène-butadiène de 50 à 80 % en poids et une viscosité dans une solution toluénique à 15 % en poids à 25°C de 20 à 40 mPas.

2. Procédé selon la revendication 1, dans lequel le composant (I) comprend moins de 70 % en poids d'un autre copolymère à blocs compatible (B) choisi dans le groupe formé par un copolymère à blocs styrène-butadiène-styrène autre que (A), et un copolymère à blocs styrène-butadiène-styrène partiellement hydrogéné dans lequel un bloc polymère à base de butadiène est partiellement hydrogéné.

3. Procédé selon la revendication 1, dans lequel l'adhésif a une viscosité à l'état fondu à 120°C de 10 000 mPas ou moins, et un point de ramollissement de 75°C ou plus.

4. Procédé selon la revendication 1, dans lequel l'adhésif est sous la forme d'un liquide ou d'un semi-liquide.

5. Procédé selon la revendication 1, dans lequel ledit film est un film de polyéthylène.

6. Adhésif selon la revendication 5, dans lequel l'adhésif est appliqué audit film.

7. Procédé selon la revendication 1, dans lequel ledit élément est une étoffe tissée, une étoffe non tissée ou du papier.

8. Produit à jeter après usage obtenu en appliquant sans contact, sur au moins l'un d'un élément d'un produit à jeter après usage et d'un film polyoléfinique, un adhésif thermofusible à une température de 130°C ou moins et en attachant l'élément au film par utilisation de pression, **caractérisé en ce que** ledit adhésif comprend un composant (I) qui est un copolymère à blocs, un composant (II) qui est un agent collant et un composant (III) qui est un plastifiant, le composant (I) étant présent en des quantités de 17 à 30 % en poids par rapport à la quantité totale des composants (I), (II) et (III), et comprenant au moins 30 % en poids de copolymère à blocs styrène-butadiène-styrène (A) ayant une teneur en styrène de 35 à 50 % en poids, une teneur en diblocs styrène-butadiène de 50 à 80 % en poids et une viscosité dans une solution toluénique à 15 % en poids à 25°C de 20 à 40 mPas.
